# FASCICULE DE BREVET EUROPEEN

(11) **EP 2 976 324 B1**
(45) Date de publication et mention de la délivrance du brevet: **03.04.2019**
(21) Numéro de dépôt: 14711810.3
(22) Date de dépôt: 18.02.2014
(51) Int. Cl.: C07C 309/65, H01M 10/0569

(54) **COMPOSES SULFONATES SPECIFIQUES UTILISABLES COMME SOLVANT D'ELECTROLYTE POUR BATTERIES AU LITHIUM**
ALS ELEKTROLYTLÖSUNGSMITTEL FÜR LITHIUMBATTERIEN VERWENDBARE SPEZIFISCHE SULFONATVERBINDUNGEN
SPECIFIC SULFONATE COMPOUNDS THAT CAN BE USED AS ELECTROLYTE SOLVENT FOR LITHIUM BATTERIES

(30) Priorité: 20.03.2013 FR 1352493
(43) Date de publication de la demande: 27.01.2016
(73) Titulaire: Renault S.A.S., 92100 Boulogne-Billancourt (FR)
(72) Inventeur: CADRA, Stéphane, F-37550 Saint Avertin (FR); BEORD, Charlotte, F-37550 Saint Avertin (FR); PIERRE, Nathalie, F-37260 Monts (FR); GALIANO, Hervé, F-37700 La Ville aux Dames (FR)
(74) Mandataire: Brevalex
(86) Numéro de dépôt international: PCT/FR2014/050339
(87) Numéro de publication internationale: WO 2014/154963

(56) Documents cités:
- EP-A1- 2 351 735
- CN-A- 102 532 228
- JP-A- 2007 073 318
- JP-A- 2012 190 700
- US-A1- 2006 286 459
- CLAÚDIA&EMSP14;C. CASSOL ET AL: "A Simple and Practical Method for the Preparation and Purity Determination of Halide-Free Imidazolium Ionic Liquids", ADVANCED SYNTHESIS & CATALYSIS, vol. 348, no. 1-2, 1 janvier 2006 (2006-01-01), pages 243-248, XP055012226, ISSN: 1615-4150, DOI: 10.1002/adsc.200505295
- SUN X G ET AL: "New sulfone electrolytes for rechargeable lithium batteries", ELECTROCHEMISTRY COMMUNICATIONS, ELSEVIER, AMSTERDAM, NL, vol. 7, no. 3, 1 mars 2005 (2005-03-01), pages 261-266, XP027706394, ISSN: 1388-2481 [extrait le 2005-03-01]

## Description

### DOMAINE TECHNIQUE

La présente invention a trait à des compositions comprenant des composés sulfonates spécifiques, l'utilisation de ces composés en tant que solvants aptes, en particulier, à permettre la dissolution de sels de lithium.

C'est donc ainsi tout naturellement que ces composés peuvent trouver application dans le domaine des électrolytes, et notamment des électrolytes destinés à entrer dans la constitution des batteries au lithium.

Les batteries au lithium sont particulièrement intéressantes pour les domaines où l'autonomie est un critère primordial, tels que cela est le cas des domaines de l'informatique, de la vidéo, de la téléphonie mobile, des transports tels que les véhicules électriques, les véhicules hybrides, ou encore des domaines médicaux, spatiaux, de la microélectronique.

D'un point de vue fonctionnel, les batteries au lithium reposent sur le principe de l'intercalation-désintercalation du lithium au sein des matériaux constitutifs des électrodes des cellules électrochimiques de la batterie.

Plus précisément, la réaction à l'origine de la production de courant (c'est-à-dire lorsque la batterie est en mode de décharge) met en jeu le transfert, par l'intermédiaire d'un électrolyte conducteur d'ions lithium, de cations lithium provenant d'une électrode négative qui viennent s'intercaler dans le réseau accepteur de l'électrode positive, tandis que des électrons issus de la réaction à l'électrode négative vont alimenter le circuit extérieur, auquel sont reliées les électrode positive et négative.

Ces électrolytes peuvent consister en un mélange comprenant au moins un solvant organique et au moins un sel de lithium pour assurer la conduction desdits ions lithium, ce qui nécessite que le sel de lithium soit dissous dans ledit solvant organique.

Ce solvant organique peut être un solvant appartenant à la famille des carbonates, des carboxylates, des éthers linéaires ou cycliques, auquel il est adjoint des additifs :
- comme des composés sultones en combinaison avec des carbonates de vinylène comme décrit dans US 2004/0137333, destinés à protéger les anodes carbonées non graphitisable de la formation, à leur surface, de protubérances ;
- comme des composés mononitriles ou dinitriles en combinaison avec des composés comprenant un groupe -S=O comme décrit dans US 2004/0013946 destinés à diminuer les phénomènes d'érosion des éléments internes métalliques compris dans une batterie ;
- comme des composés esters sulfonates cycliques comprenant deux groupes sulfonyles ou des composés esters sulfonates linéaires comprenant deux groupes sulfonyles comme décrit dans US 2010/0062332, destinés à protéger l'électrode négative en graphite des phénomènes de dépôt de composé lithié lors de la première charge ;
- comme des composés (di)-*tert*-butylphényl-alkylsulfonate ou (di)*-tert-*butylphénylarylsulfonates comme décrit dans US 2010/0055576, en vue d'améliorer les performances de cyclage d'une batterie au lithium ;
- comme des composés siloxanes en combinaison avec des composés sulfonates comprenant un groupe 1,3-dioxane comme décrit dans US 2012/0034532, destinés à limiter les phénomènes de dégradation de la capacité de batteries utilisés à long terme.

Ce solvant organique peut également être un solvant seul tels que les sulfones décrites par Sun, x, Austen angell C, Electrochemistry communications, 7, 2005, 261-266*.*

Comme il ressort de ce qui précède, les électrolytes peuvent être de nature relativement complexe, dans la mesure où ils impliquent, outre la présence d'un ou plusieurs solvants organiques et d'un ou plusieurs sels de lithium, la présence d'un ou plusieurs additifs.

Les inventeurs de la présente invention se sont ainsi proposé de mettre au point des compositions comprenant de nouveaux composés qui présentent les caractéristiques suivantes :
- des composés qui peuvent être utilisés comme solvants pour entrer dans la constitution d'électrolytes pour batteries au lithium ;
- des composés qui présentent des propriétés adaptées pour la constitution d'électrolyte en termes de viscosité (par exemple, une viscosité inférieure à 10 mPa.s), de constante diélectrique (par exemple, supérieure à 20), de conductivité (par exemple, une conductivité supérieure à 1 mS/cm) tout en étant stables pour les hauts potentiels de fonctionnement (Eₒₓ> 5V vs Li);
- des composés, qui permettent de limiter voire de supprimer la présence d'additifs d'électrolytes ;
- des composés qui, une fois associés avec un sel de lithium, tel que LiPF₆, permettent d'obtenir des électrolytes aux propriétés intéressantes (telles qu'une conductivité supérieure à 1 mS/cm, une température de fusion inférieure à -20°C).

### EXPOSÉ DE L'INVENTION

Ainsi, l'invention a trait à des compositions constituées exclusivement d'au moins un composé sulfonate de formule (I) suivante : 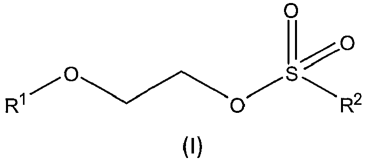 dans laquelle :
- R² représente un groupe éthyle, un groupe n-propyle ou un groupe isopropyle ;
- lorsque R² est un groupe éthyle, R¹ est un groupe éthyle, un groupe n-propyle ou un groupe n-butyle ;
- lorsque R² est un groupe n-propyle, R¹ est un groupe méthyle, un groupe éthyle, un groupe n-propyle ou un groupe n-butyle ; ou
- lorsque R² est un groupe isopropyle, R¹ est un groupe méthyle, un groupe éthyle, un groupe n-propyle ou un groupe n-butyle ; et
d'au moins un sel de lithium.

Des composés répondant à cette spécificité sont les composés suivants :
- un composé pour lequel R² est un groupe n-propyle et R¹ est un groupe méthyle, ce composé répondant à la formule (II) suivante : 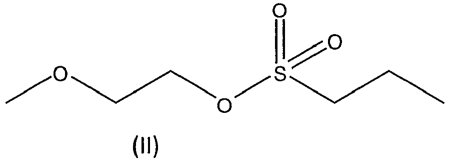
- un composé pour lequel R² est un groupe n-propyle et R¹ est un groupe éthyle, ce composé répondant à la formule (III) suivante : 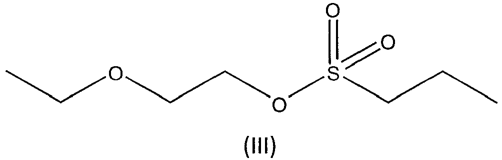
- un composé pour lequel R² est un groupe éthyle et R¹ est un groupe éthyle, ce composé répondant à la formule (IV) suivante :

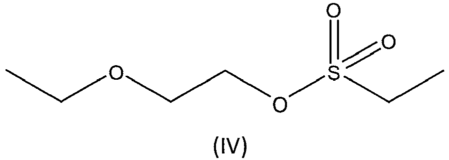

Les composés sulfonates entrant dans les compositions de l'invention peuvent être préparés par la mise en oeuvre d'un procédé comprenant une étape de réaction entre un composé hydroxyéther de formule R¹-O-CH₂-CH₂-OH, R¹ étant tel que défini ci-dessus avec un composé X-SO₂-R², avec X étant un atome d'halogène tel que du chlore, et R² étant tel que défini ci-dessus, dans un milieu comprenant au moins une base et un solvant organique, plus spécifiquement un solvant organique aprotique.

Ladite base peut être :
- un composé amine, que la triméthylamine, la triéthylamine, la tri(n-butyl)amine ;
- un composé pyridine, tel que la pyridine ; ou
- un composé imidazole, tel que l'imidazole.

Ledit solvant organique peut être un solvant hydrocarbure aliphatique, un solvant aromatique ou un solvant éther cyclique ou acyclique.

La réaction entre le composé alcoolique de formule R¹-O-CH₂-CH₂-OH et le composé de formule X-SO₂-R² s'effectue, classiquement, à température ambiante, après une mise en contact de ces composés à une température sub-ambiante.

A l'issue de cette étape de réaction, le procédé de préparation des composés entrant dans les compositions de l'invention peut comprendre une étape de purification du composé obtenu, cette étape de purification pouvant consister en :
- une étape de volatilisation des composés volatils suivie d'une étape de distillation à pression réduite ; ou
- une étape de purification par chromatographie sur gel de silice.

Les composés entrant dans les compositions de l'invention présentent, entre autres propriétés, un potentiel d'oxydation supérieur à 5 V par rapport au couple Li⁺/Li, une viscosité faible (inférieure à 10 mPa.s), une constante diélectrique élevée (supérieure à 20) et une température de fusion sub-ambiante. Qui plus est, les composés de l'invention présentent une bonne capacité à solubiliser des sels de lithium.

C'est donc tout naturellement qu'ils trouvent leur application comme solvant organique d'électrolyte, et plus spécifiquement comme solvant organique pouvant entrer dans la constitution d'un électrolyte comprenant au moins un sel de lithium destiné à une batterie au lithium.

Ainsi, en résumé, l'invention a trait à :
- l'utilisation d'un composé tel que défini ci-dessus comme solvant organique d'au moins un sel de lithium, en particulier comme solvant organique d'au moins un sel de lithium dans un électrolyte conducteur d'ions lithium ;
- une composition, plus spécifiquement une composition liquide, qui peut être un électrolyte conducteur d'ions lithium, constitué exclusivement d'au moins un composé tel que défini ci-dessus et au moins un sel de lithium ; et
- une batterie au lithium comprenant au moins une cellule électrochimique comprenant un électrolyte conducteur d'ions lithium tel que défini ci-dessus disposé entre une électrode positive et une électrode négative; et
- des composés sulfonates spécifiques de formule (I) suivante : 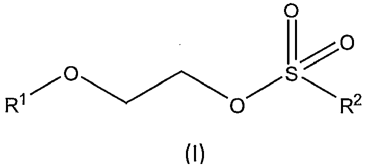 dans laquelle :
   - R² représente un groupe éthyle, un groupe n-propyle ou un groupe isopropyle ;
   - lorsque R² est un groupe éthyle, R¹ est un groupe n-propyle ou un groupe n-butyle ;
   - lorsque R² est un groupe n-propyle, R¹ est un groupe éthyle, un groupe n-propyle ou un groupe n-butyle ; ou
   - lorsque R² est un groupe isopropyle, R¹ est un groupe éthyle, un groupe n-propyle ou un groupe n-butyle ; et
   en particulier, un composé sulfonate, pour lequel R² est un groupe n-propyle et R¹ est un groupe éthyle.

A titre d'exemples, le sel de lithium peut être choisi dans le groupe constitué par LiPF₆, LiClO₄, LiBF₄, LiAsF₆, LiCF₃SO₃, LiN(CF₃SO₂)₃, LiN(C₂F₅SO₂), le bistrifluorométhylsulfonylimide de lithium (connu sous l'abréviation LiTFSI) LiN[SO₂CF₃]₂ et les mélanges de ceux-ci. Plus spécifiquement, le sel de lithium peut LiPF₆ ou LiTFSI.

La composition susmentionnée est composée exclusivement d'au moins un composé conforme à l'invention et d'au moins un sel de lithium.

Dans la batterie au lithium, l'électrolyte liquide susmentionné peut être amené, dans les cellules électrochimiques des batteries au lithium, à imprégner un séparateur, lequel est disposé entre l'électrode positive et l'électrode négative de la cellule électrochimique.

Ce séparateur peut être en un matériau poreux, tel qu'un matériau polymérique, apte à accueillir dans sa porosité l'électrolyte liquide. Plus spécifiquement, il peut s'agir d'une membrane de type Celguard 2400.

Par électrode positive, on entend, classiquement, dans ce qui précède et ce qui suit, l'électrode qui fait office de cathode, quand le générateur débite du courant (c'est-à-dire lorsqu'il est en processus de décharge) et qui fait office d'anode lorsque le générateur est en processus de charge.

Par électrode négative, on entend, classiquement, dans ce qui précède et ce qui suit, l'électrode qui fait office d'anode, quand le générateur débite du courant (c'est-à-dire lorsqu'il est en processus de décharge) et qui fait office de cathode, lorsque le générateur est en processus de charge.

Avantageusement, l'électrode négative peut être en un matériau à base d'un oxyde lithié de titane, tel que Li₄Ti₅O₁₂, qui constitue le matériau d'insertion de lithium, lequel oxyde peut être dispersé dans un liant polymérique, par exemple, un liant polyfluorure de vinylidène.

L'électrode positive, quant à elle, peut être en un matériau à base d'un oxyde de métal de transition lithié (le métal pouvant être, par exemple, du nickel, du manganèse), plus spécifiquement LiMn_{2-z}Ni_{z}O₄ (avec 0<z<2), lequel oxyde peut être dispersé dans un liant polymérique, par exemple, un liant polyfluorure de vinylidène.

Que ce soit pour l'électrode négative ou l'électrode positive, chacune d'entre elles est, avantageusement, associée à un collecteur de courant métallique, par exemple, un collecteur de courant en aluminium.

L'invention va maintenant être décrite, en référence aux exemples suivants, donnés à titre indicatif et non limitatif.

### EXPOSÉ DÉTAILLÉ DE MODES DE RÉALISATION PARTICULIERS

### EXEMPLE 1

Cet exemple illustre la préparation d'un composé utilisable dans les compositions de l'invention : le 2-méthoxyéthyl propane-1-sulfonate, cette préparation pouvant être illustrée par le schéma réactionnel suivant :

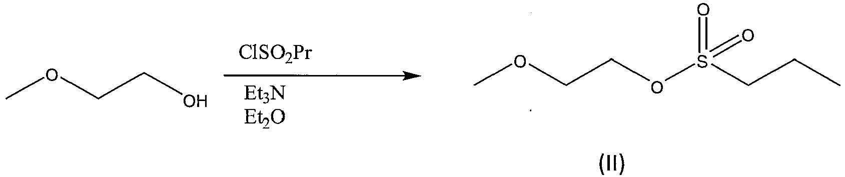

Dans un tricol de 50 mL purgé à l'argon sont introduits 20 mL d'éther anhydre, 1,6 mL (20 mmol) de méthoxyéthanol et 4,2 mL (30 mmol) de triéthylamine. L'ensemble est homogénéisé et refroidi à 0°C (bain de glace). 2,25 mL (20 mmol) de chlorure de propanesulfonyle sont introduits au goutte à goutte à l'aide d'une seringue, ce qui entraîne une légère augmentation de température du mélange, du fait du caractère exothermique de la réaction. Quelques minutes après l'ajout, le bain de glace est retiré et l'agitation est poursuivie 24 heures à température ambiante.

En fin de réaction, le mélange est filtré et le filtrat est extrait avec une solution d'acide chlorhydrique 1M. La phase organique est récupérée, séchée et évaporée (sous 400 mbars). Le résidu est distillé à pression réduite. Le produit résultant est un liquide incolore (Rendement de 67%).

Le produit a été analysé par RMN ¹H et RMN ¹³C et correspond au composé de formule (II) susmentionnée.

Les résultats sont les suivants :
RMN ¹H (CDCl₃) : 1,00 (t, 3H) ; 1,82 (sext, 2H) ; 3,05 (t, 2H) ; 3,32 (s, 3H); 3,57 (t, 2H); 4,26 (t, 2H).
RMN ¹³C (CDCl₃): 12,79; 17,13; 52,18; 58,94 ; 68,53 ; 70,40.

Ce composé répond aux propriétés suivantes :
- Point de fusion : inférieure à -90°C.
- Température d'ébullition : 280°C
- Viscosité : 6,78 mPa.s ;
- Constante diélectrique : 32.

Lorsque qu'un électrolyte est formé par dissolution de LiPF₆ (1,52 g, 10 mmol) dans 10 mL du composé sulfonate susmentionné sous atmosphère inerte dans une boîte à gants suivi d'une agitation jusqu'à complète dissolution du sel, un point de fusion très inférieur à -20°C (plus précisément, le point de fusion est de l'ordre de -80°C) est mesuré pour l'électrolyte obtenu, ce qui dénote d'excellentes propriétés antigel. Il est obtenu également une conductivité de 1,26 mS/cm et une viscosité de 34,2 mPa.s, toutes deux mesurées à 20°C. Ceci atteste de l'aptitude des compositions de l'invention à présenter une conductivité importante, tout en présentant une viscosité importante.

Lorsque qu'un électrolyte est formé par dissolution de LiN(SO₂CF₃)₂ (symbolisé par LiTFSI) (2,87 g, 10 mmol) dans 10 mL du composé sulfonate susmentionné sous atmosphère inerte dans une boîte à gants suivi d'une agitation jusqu'à complète dissolution du sel, un point de fusion très inférieur à -20°C (plus précisément, le point de fusion est de l'ordre de -80°C) est mesuré pour l'électrolyte obtenu, ce qui dénote d'excellentes propriétés antigel. Il est obtenu également une conductivité de 1,17 mS/cm et une viscosité de 23,5 mPa.s, toutes deux mesurées à 20°C.

### EXEMPLE 2

Cet exemple illustre la préparation d'un composé conforme à l'invention : le 2-éthoxyéthyl propane-1-sulfonate, cette préparation pouvant être illustrée par le schéma réactionnel suivant :

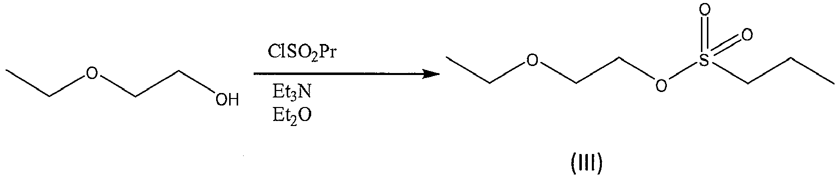

Dans un tricol de 50 mL purgé à l'argon sont introduits 20 mL d'éther anhydre, 2,0 mL (20 mmol) d'éthoxyéthanol et 4,2 mL (30 mmol) de triéthylamine. L'ensemble est homogénéisé et refroidi à 0°C (bain de glace). 2,25 mL (20 mmol) de chlorure de propanesulfonyle sont introduits au goutte à goutte à l'aide d'une seringue, ce qui entraîne une légère augmentation de température du mélange, du fait du caractère exothermique de la réaction. Quelques minutes après l'ajout, le bain de glace est retiré et l'agitation est poursuivie 24 heures à température ambiante.

En fin de réaction, le mélange est filtré et le filtrat est extrait avec une solution d'acide chlorhydrique 1M. La phase organique est récupérée, séchée et évaporée (sous 400 mbars). Le résidu est distillé à pression réduite. Le produit résultant est un liquide translucide (Rendement : 81%).

Le produit a été analysé par RMN ¹H et RMN ¹³C.

Les résultats sont les suivants :
RMN ¹H (CDCl₃) : 1,05 (t, 3H) ; 1,18 (t, 3H) ; 1,89 (sext, 2H); 3,11 (t, 2H) ; 3,52 (quad, 2H) ; 3,66 (t, 2H) ; 4,32(t, 2H).
RMN ¹³C (CDCl₃): 12,69; 14,89; 17,08; 52,02; 66,61 ; 68,21 ; 68,86.
Ce composé répond aux propriétés suivantes :
- Point de fusion : inférieur à -90°C ;
- Température d'ébullition : 290°C ;
- Viscosité (mPa.s) : 6,52 ;
- Constante diélectrique : 32.

### EXEMPLE 3

Cet exemple illustre la préparation d'un composé utilisable dans les compositions de l'invention : le 2-éthoxyéthyl éthane-1-sulfonate, cette préparation pouvant être illustrée par le schéma réactionnel suivant :

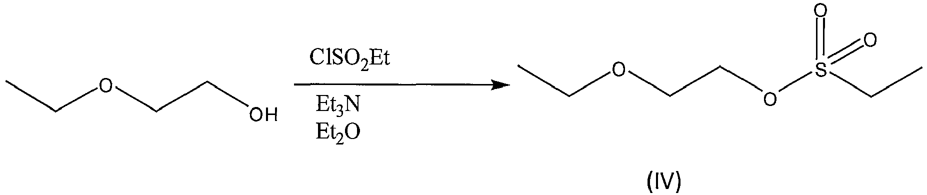

Dans un tricol de 50 mL purgé à l'argon sont introduits 20 mL d'éther anhydre, 2,0 mL (20 mmol) d'éthoxyéthanol et 4,2 mL (30 mmol) de triéthylamine. L'ensemble est homogénéisé et refroidi à 0°C (bain de glace). 2,25 mL (20 mmol) de chlorure d' éthanesulfonyle sont introduits au goutte à goutte à l'aide d'une seringue, ce qui entraîne une légère augmentation de température du mélange, du fait du caractère exothermique de la réaction. Quelques minutes après l'ajout, le bain de glace est retiré et l'agitation est poursuivie 24 heures à température ambiante.

En fin de réaction, le mélange est filtré et le filtrat est extrait avec une solution d'acide chlorhydrique 1M. La phase organique est récupérée, séchée et évaporée (sous 400 mbars). Le résidu est distillé à pression réduite. Le produit récupéré est un liquide translucide (Rendement : 64%)
Le produit a été analysé par RMN ¹H et RMN ¹³C et correspond au composé de formule (IV) susmentionnée.

Les résultats sont les suivants :
RMN ¹H (CDCl₃) : 1,09 (t, 3H) ; 1,30 (t, 3H) ; 3,08 (quad, 2H) ; 3,44 (quad, 2H) ; 3,58 (t, 2H) ; 4,23 (t, 2H).
RMN ¹³C (CDCl₃): 9,28; 13,02 ; 17,50; 26,89; 53,25; 85,48.

Ce composé répond aux propriétés suivantes :
- Point de fusion : inférieure à -90°C ;
- Température d'ébullition : 280°C
- Viscosité (mPa.s) : 7,3 ;
- Constante diélectrique : 40.

Lorsque qu'un électrolyte est formé par dissolution de LiPF₆ (1,52 g, 10 mmol) dans 10 mL du composé sulfonate susmentionné sous atmosphère inerte dans une boîte à gants suivi d'une agitation jusqu'à complète dissolution du sel, un point de fusion très inférieur à -20°C (plus précisément, le point de fusion est inférieur à -80°C) est mesuré pour l'électrolyte obtenu, ce qui dénote d'excellentes propriétés antigel. Il est obtenu également une conductivité de 1,23 mS/cm et une viscosité de 34 mPa.s, toutes deux mesurées à 20°C.

Lorsque qu'un électrolyte est formé par dissolution de LiN(SO₂CF₃)₂ (symbolisé par LiTFSI) (2,87 g, 10 mmol) dans 10 mL du composé sulfonate susmentionné sous atmosphère inerte dans une boîte à gants suivi d'une agitation jusqu'à complète dissolution du sel, un point de fusion très inférieur à -20°C (plus précisément, le point de fusion est inférieur à -80°C) est mesuré pour l'électrolyte obtenu, ce qui dénote d'excellentes propriétés antigel. Il est obtenu également une conductivité de 1,14 mS/cm et une viscosité de 25,5 mPa.s, toutes deux mesurées à 20°C.

## Revendications

1. Composition constituée exclusivement d'au moins un composé sulfonate de formule (I) suivante : 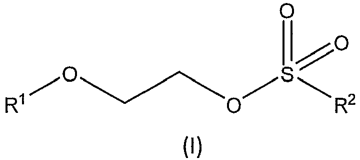 dans laquelle :
- R² représente un groupe éthyle, un groupe n-propyle ou un groupe isopropyle ;
- lorsque R² est un groupe éthyle, R¹ est un groupe éthyle, un groupe n-propyle ou un groupe n-butyle ;
- lorsque R² est un groupe n-propyle, R¹ est un groupe méthyle, un groupe éthyle, un groupe n-propyle ou un groupe n-butyle ; ou
- lorsque R² est un groupe isopropyle, R¹ est un groupe méthyle, un groupe éthyle, un groupe n-propyle ou un groupe n-butyle ; et
d'au moins un sel de lithium.

2. Composition selon la revendication 1, dans laquelle R² est un groupe n-propyle et R¹ est un groupe méthyle.

3. Composition selon la revendication 1, dans laquelle R² est un groupe n-propyle et R¹ est un groupe éthyle.

4. Composition selon la revendication 1, dans laquelle R² est un groupe éthyle et R¹ est un groupe éthyle.

5. Composition selon la revendication l'une quelconque des revendications précédentes, dans lequel le sel de lithium est choisi dans le groupe constitué par LiPF₆, LiClO₄, LiBF₄, LiAsF₆, LiCF₃SO₃, LiN(CF₃SO₂)₃, LiN(C₂F₅SO₂), le bistrifluorométhylsulfonylimide de lithium LiN[SO₂CF₃]₂ et les mélanges de ceux-ci.

6. Composition selon l'une quelconque des revendications précédentes, qui est un électrolyte conducteur d'ions lithium.

7. Utilisation d'un composé sulfonate de formule (I) suivante 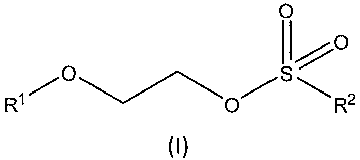 dans laquelle :
- R² représente un groupe éthyle, un groupe n-propyle ou un groupe isopropyle ;
- lorsque R² est un groupe éthyle, R¹ est un groupe éthyle, un groupe n-propyle ou un groupe n-butyle ;
- lorsque R² est un groupe n-propyle, R¹ est un groupe méthyle, un groupe éthyle, un groupe n-propyle ou un groupe n-butyle ; ou
- lorsque R² est un groupe isopropyle, R¹ est un groupe méthyle, un groupe éthyle, un groupe n-propyle ou un groupe n-butyle ;
comme solvant organique d'au moins un sel de lithium.

8. Batterie au lithium comprenant au moins une cellule électrochimique comprenant un électrolyte tel que défini à la revendication 6 disposé entre une électrode positive et une électrode négative.

9. Batterie au lithium selon la revendication 8, dans laquelle l'électrode négative est en un matériau à base d'un oxyde lithié de titane, tel que Li₄Ti₅O₁₂.

10. Batterie au lithium selon la revendication 8 ou 9, dans laquelle l'électrode positive est en un matériau à base d'un matériau de formule LiMn_{2-z}Ni_{z}O₄, avec 0<z<2.

11. Composé sulfonate de formule (I) suivante : 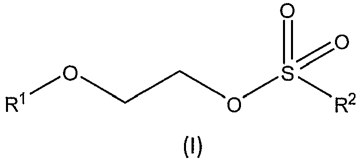 dans laquelle :
- R² représente un groupe éthyle, un groupe n-propyle ou un groupe isopropyle ;
- lorsque R² est un groupe éthyle, R¹ est un groupe n-propyle ou un groupe n-butyle ;
- lorsque R² est un groupe n-propyle, R¹ est un groupe éthyle, un groupe n-propyle ou un groupe n-butyle ; ou
- lorsque R² est un groupe isopropyle, R¹ est un groupe éthyle, un groupe n-propyle ou un groupe n-butyle.

12. Composé selon la revendication 11, dans lequel R² est un groupe n-propyle et R¹ est un groupe éthyle.

## Patentansprüche

1. Zusammensetzung, welche ausschließlich aus wenigstens einer Sulfonat-Verbindung der folgenden Formel (I) besteht: 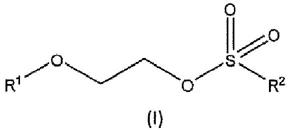 wobei gilt:
- R² steht für eine Ethylgruppe, eine n-Propylgruppe oder eine Isopropylgruppe;
- wenn R² eine Ethylgruppe ist, ist R¹ eine Ethylgruppe, eine n-Propylgruppe oder eine n-Butylgruppe;
- wenn R² eine n-Propylgruppe ist, ist R¹ eine Methylgruppe, eine Ethylgruppe, eine n-Propylgruppe oder eine n-Butylgruppe; oder
- wenn R² eine Isopropylgruppe ist, ist R¹ eine Methylgruppe, eine Ethylgruppe, eine n-Propylgruppe oder eine n-Butylgruppe; und
wenigstens ein Lithiumsalz.

2. Zusammensetzung nach Anspruch 1, bei welcher R² eine n-Propylgruppe und R¹ eine Methylgruppe ist.

3. Zusammensetzung nach Anspruch 1, bei welcher R² eine n-Propylgruppe und R¹ eine Ethylgruppe ist.

4. Zusammensetzung nach Anspruch 1, bei welcher R² eine Ethylgruppe und R¹ eine Ethylgruppe ist.

5. Zusammensetzung nach einem der vorhergehenden Ansprüche, bei welcher das Lithiumsalz aus der Gruppe gewählt ist, welche LiPF₆, LiClO₄, LiBF₄, LiAsF₆, LiCF₃SO₃, LiN(CF₃SO₂)₃, LiN(C₂F₅SO₂), Lithium-Bistrifluormethylsulfonylimid LiN[SO₂CF₃]₃ und deren Mischungen umfasst.

6. Zusammensetzung nach einem der vorhergehenden Ansprüche, welche ein Lithiumionen leitender Elektrolyt ist.

7. Verwendung einer Sulfonat-Verbindung der folgenden Formel (I): 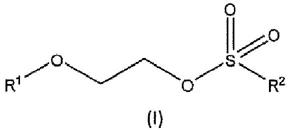 wobei gilt:
- R² steht für eine Ethylgruppe, eine n-Propylgruppe oder eine Isopropylgruppe;
- wenn R² eine Ethylgruppe ist, ist R¹ eine Ethylgruppe, eine n-Propylgruppe oder eine n-Butylgruppe;
- wenn R² eine n-Propylgruppe ist, ist R¹ eine Methylgruppe, eine Ethylgruppe, eine n-Propylgruppe oder eine n-Butylgruppe; oder
- wenn R² eine Isopropylgruppe ist, ist R¹ eine Methylgruppe, eine Ethylgruppe, eine n-Propylgruppe oder eine n-Butylgruppe;
als organisches Lösungsmittel wenigstens eines Lithiumsalzes.

8. Lithiumbatterie, welche wenigstens eine elektrochemische Zelle umfasst, die einen zwischen einer positiven Elektrode und einer negativen Elektrode angeordneten Elektrolyten gemäß der Definition in Anspruch 6 umfasst.

9. Lithiumbatterie nach Anspruch 8, bei welcher die negative Elektrode ein Material auf der Basis eines lithiumhaltigen Titanoxids ist, wie beispielsweise Li₄Ti₅O₁₂.

10. Lithiumbatterie nach Anspruch 8 oder 9, bei welcher die positive Elektrode aus einem Material auf der Basis eines Materials mit der Formel LiMn_{2-z}Ni_{Z}O₄ besteht, mit 0<z<2.

11. Sulfonat-Verbindung der folgenden Formel (I) besteht: 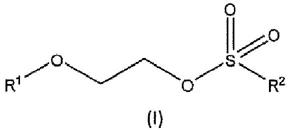 wobei gilt:
- R² steht für eine Ethylgruppe, eine n-Propylgruppe oder eine Isopropylgruppe;
- wenn R² eine Ethylgruppe ist, ist R¹ eine n-Propylgruppe oder eine n-Butylgruppe;
- wenn R² eine n-Propylgruppe ist, ist R¹ eine Ethylgruppe, eine n-Propylgruppe oder eine n-Butylgruppe; oder
- wenn R² eine Isopropylgruppe ist, ist R¹ eine Ethylgruppe, eine n-Propylgruppe oder eine n-Butylgruppe.

12. Verbindung nach Anspruch 11, bei welcher R² eine n-Propylgruppe ist und R¹ eine Ethylgruppe ist.

## Claims

1. Composition composed exclusively of at least one sulfonate compound with the following formula (I): 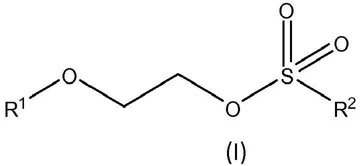 in which:
- R² represents an ethyl group, an n-propyl group or an isopropyl group;
- when R² is an ethyl group, R¹ is an ethyl group, an n-propyl group or an n-butyl group;
- when R² is an n-propyl group, R¹ is a methyl group, an ethyl group, an n-propyl group or an n-butyl group; or
- when R² is an isopropyl group, R¹ is a methyl group, an ethyl group, an n-propyl group or an n-butyl group; and
at least one lithium salt.

2. Composition according to claim 1, in which R² is an n-propyl group and R¹ is a methyl group.

3. Composition according to claim 1, in which R² is an n-propyl group and R¹ is an ethyl group.

4. Composition according to claim 1, in which R² is an ethyl group and R¹ is an ethyl group.

5. Composition according to any of the preceding claims, in which the lithium salt is chosen from the group composed of LiPF₆, LiClO₄, LiBF₄, LiAsF₆, LiCF₃SO₃, LiN(CF₃SO₂)₃, LiN(C₂F₅SO₂), lithium bistrifluoromethylsulfonylimide LiN[SO₂CF₃]₂ and mixtures of them.

6. Composition according to any of the preceding claims, that is a lithium ion conducting electrolyte.

7. Use of a sulfonate compound with the following formula (I): 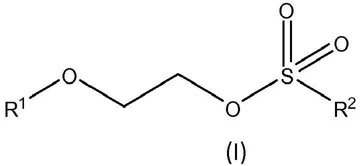 in which:
- R² represents an ethyl group, an n-propyl group or an isopropyl group;
- when R² is an ethyl group, R¹ is an ethyl group, an n-propyl group or an n-butyl group;
- when R² is an n-propyl group, R¹ is a methyl group, an ethyl group, an n-propyl group or an n-butyl group; or
- when R² is an isopropyl group, R¹ is a methyl group, an ethyl group, an n-propyl group or an n-butyl group;
as an organic solvent of at least one lithium salt.

8. Lithium battery comprising at least one electrochemical cell comprising an electrolyte like that defined in claim 6 located between a positive electrode and a negative electrode.

9. Lithium battery according to claim 8, in which the negative electrode is a material based on a lithium titanium oxide such as Li₄Ti₅O₁₂.

10. Lithium battery according to claim 8 or 9, in which the positive electrode is a material based on a material with formula LiMn_{2-z}Ni_{z}O₄, where 0<z<2.

11. Sulfonate compound with the following formula (I): 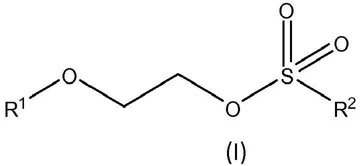 in which:
- R² represents an ethyl group, an n-propyl group or an isopropyl group;
- when R² is an ethyl group, R¹ is an n-propyl group or an n-butyl group;
- when R² is an n-propyl group, R¹ is an ethyl group, an n-propyl group or an n-butyl group; or
- when R² is an isopropyl group, R¹ is an ethyl group, an n-propyl group or an n-butyl group.

12. Sulfonate compound according to claim 11, in which R² is an n-propyl group and R¹ is an ethyl group.
